# EUROPÄISCHE PATENTSCHRIFT

(11) **EP 3 067 633 B1**
(45) Veröffentlichungstag und Bekanntmachung des Hinweises auf die Patenterteilung: **31.01.2018**
(21) Anmeldenummer: 16158222.6
(22) Anmeldetag: 13.01.2010
(51) Int. Cl.: F24F 3/16, A61L 2/20, A61L 9/16, B08B 15/02, B01D 46/00, A61L 9/015

(54) **DEKONTAMINATIONSVERFAHREN**
DECONTAMINATION METHOD
PROCEDE DE DECONTAMINATION

(43) Veröffentlichungstag der Anmeldung: 14.09.2016
(62) Teilanmeldung aus: 12179711.2
(73) Patentinhaber: Metall + Plastic GmbH, 78315 Radolfzell (DE)
(72) Erfinder: VON STENGLIN, Christoph, 78315 Radolfzell (DE); HAUK, Jürgen, 85354 Freising (DE)
(74) Vertreter: Patentanwälte Behrmann Wagner PartG mbB

(56) Entgegenhaltungen:
- EP-A1- 0 604 925
- EP-A1- 1 426 122
- WO-A1-98/50134
- WO-A1-2005/113169
- US-A- 6 010 400

## Beschreibung

Die Erfindung betrifft ein Verfahren gemäß dem Oberbegriff des Anspruchs 1 zum Entfernen von Gas- und/oder dampfförmigen Dekontaminationsmittel, insbesondere von Wasserstoffperoxiddampf, aus einem zu dekontaminierenden Raum, mittels einer Dekontaminationsanordnung (Dekontaminationsvorrichtung), insbesondere für pharmatechnische Anwendungen, umfassend einen zu dekontaminierenden Raum, insbesondere einen Isolatorraum, sowie eine zum Entziehen von Gas- und/oder dampfförmigen Dekontaminationsmittel, insbesondere Wasserstoffperoxid, aus der Raumluft ausgebildete Reinigungseinrichtung. Für den Fall der Inkludierung des Verfahrensschrittes der Zuführung von Dekontaminationsmittel in dem zu dekontaminierenden Raum zeitlich vor dem Freispülschritt handelt es sich um ein kombiniertes Dekontaminations-/Dekontaminationsmittelentfernungsverfahren.

Aus der EP 0 604 925 B1 ist ein Isolator für pharmatechnische Anwendungen bekannt. Dieser umfasst einen zu dekontaminierenden Manipulatorraum, über welchem ein sogenannter Umlufterzeugerraum, umfassend ein Umluftgebläse, angeordnet ist. Der zu dekontaminierende Raum ist mit Doppelglasscheiben ausgestattet, zwischen denen ein Strömungskanal ausgebildet ist, der es ermöglicht, dass die Raumluft während der Dekontamination zwischen dem Manipulatorraum und dem Umluftraum mit dem Umluftgebläse zirkuliert werden kann. Zur Dekontamination wird üblicherweise Wasserstoffperoxiddampf eingesetzt, der mittels eines Wasserstoffperoxidverdampfers erzeugt wird, welcher üblicherweise in einer Leitung angeordnet ist, die aus dem zu dekontaminierenden Raum ausmündet, um die mit Dekontaminationsmitteldampf anzureichernde Raumluft ansaugen zu können und in einem Bereich nach dem Verdampfer, die wieder in den zu dekontaminierenden Raum einmündet, um die mit Dekontaminationsmittel angereicherte Luft wieder in den zu dekontaminierenden Raum abgeben zu können.

Die zuvor beschrieben ausgebildeten Dekontaminationseinrichtungen weisen einen Frischluftanschluss auf, über den konditionierte (getrocknete und temperierte) Frischluft zugeführt werden kann, um das Dekontaminationsmittel aus dem zu dekontaminierenden Raum nach erfolgter Dekontamination ausblasen, d.h. den Raum spülen zu können. Dieses sogenannte Freispülen nimmt einen erheblichen Zeitraum in Anspruch, der in Abhängigkeit der Größe des Isolators und der gewünschten, zu erzielenden (minimalen) Dekontaminationsmittelkonzentration von beispielsweise 1 ppm oder weniger drei bis zehn Stunden betragen kann. Die in der Praxis zum Einsatz kommenden Frischluftkonditioniereinrichtungen umfassen ein Zuluftgebläse (Frischluftgebläse), welches üblicherweise derart ausgelegt ist, dass mit diesem in den zu dekontaminierenden Raum und allen damit strömungstechnisch verbundenen Räumen ein etwa 150facher Luftwechsel pro Stunde realisierbar ist. Es ist also etwa ein 150faches Raumvolumen pro Stunde mittels des Frischluftgebläses zuführbar.

Häufig sind bekannte Dekontaminationsanordnungen im Abluftkanal mit einer katalytisch wirkenden Reinigungseinrichtung versehen, die die in die Umgebung abgelassene Raumluft während des Freispülens des zu dekontaminierenden Raumes zum Schutze der Umwelt von Dekontaminationsmittel befreit. Die Reinigungseinrichtung besteht dabei im Wesentlichen aus einem Katalysator, welcher wiederum aus als Schüttgut vorliegenden MnO₂-Kugeln besteht.

Nachteilig bei den bekannten Dekontaminationsanordnungen ist die lange Freispülzeit (Reinigungszeit), die benötigt wird, um die Dekontaminationsmittelkonzentration in der Raumluft auf das gewünschte Maß zu reduzieren.

Aus der US 6,010,400 A ist eine Dekontaminationsanordnung mit einem speziellen Aufbau bekannt. Dieser umfasst eine kombinierte Zu- und Abluftleitung sowie ein Umluftgebläse, welches nicht wie bei Standarddekontaminationsanordnungen in einem Umlufterzeugerraum oberhalb des zu dekontaminierenden Raums angeordnet ist, sondern in einem Umluftrohr, welches einen unteren Bereich des zu dekontaminierenden Raumes mit einem oberen Bereich des zu dekontaminierenden Raumes verbindet. In der in Fig. 5 der US-Schrift gezeigten Ausführungsform kann der mittels des Gebläses in einem Bereich seitlich des zu dekontaminierenden Isolatorraums erzeugte Umluftstrom gelenkt werden, und zwar in einer ersten Einstellung während der Dekontamination an dem Katalysator getränkten Hepa-Filter vorbei und zu Freispülzwecken durch den Katalysator getränkten Hepa-Filter hindurch. Bei der bekannten Dekontaminationsanordnung wird das Gebläse zur Verteilung des Dekontaminationsmittels benötigt. Die gezeigte Anordnung ist nicht für den Einsatz bei Standarddekontaminationsanordnungen geeignet, bei denen der Umluftstrom nicht über neben dem Isolatorraum verlaufenden Rohrleitungen geführt ist, sondern durch durchsichtige Flachkanäle, die außen von der (durchsichtigen) Isolatorwand begrenzt sind, wie dies in der EP 0 604 925 B1 gezeigt ist.

Ausgehend von den zuvor beschriebenen Stand der Technik liegt der Erfindung die Aufgabe zugrunde, ein verbessertes Verfahren zum Betreiben einer Dekontaminationsanordnung anzugeben, mit welchem das Reduzieren der Dekontaminationsmittelkonzentration in der Raumluft in einer verkürzten Zeitspanne möglich ist. Bevorzugt soll gleichzeitig der Energieverbrauch gesenkt werden.

Hinsichtlich des Verfahrens wird die Aufgabe mit den Merkmalen des Anspruchs 1 gelöst.

Vorteilhafte Weiterbildungen der Erfindung sind in den Unteransprüchen angegeben. In den Rahmen der Erfindung fallen sämtliche Kombinationen aus zumindest zwei von in der Beschreibung, den Ansprüchen und/oder den Figuren angegebenen Merkmalen. Zur Vermeidung von Wiederholungen sollen vorrichtungsgemäß offenbarte Merkmale als verfahrensgemäß offenbart gelten und beanspruchbar sein. Ebenso sollen verfahrensgemäß offenbarte Merkmale als vorrichtungsgemäß offenbart gelten und beanspruchbar sein.

Die Erfindung führt auf ein Verfahren zum Entfernen (Absenken der Konzentration) von gas- und/oder dampfförmigem Dekontaminationsmittel, insbesondere von Wasserstoffperoxiddampf, aus einem zu dekontaminierenden Raum, unter Einsatz einer wie zuvor beschriebenen Dekontaminationsanordnung (Dekontaminationsvorrichtung). Kern der Erfindung ist es, den kontaminierenden Raum nicht oder nicht nur mit Frischluft freizuspülen, sondern durch Fördern der mit Dekontaminationsmittel belasteten Raumluft im Kreislauf durch eine Reinigungseinrichtung, die derart ausgebildet ist, dass diese, bevorzugt katalytisch, die Raumluft von Dekontaminationsmittel, insbesondere zumindest weitgehend, befreit.

Erfindungsgemäß ist vorgesehen, dass das, insbesondere als Umluftgebläse zur Erzeugung einer laminaren Luftströmung während des Betriebs zu dekontaminierenden Raums ausgebildete, Gebläse, zumindest über den größten Zeitabschnitt, während einer Konditionierungsphase, vorzugsweise während der gesamten Konditionierungsphase, während derer der zu dekontaminierende Raum mit Dekontaminationsmittel angereichert wird, ausgeschaltet ist oder bleibt. Das gleiche gilt während der Einwirkphase, also der eigentlichen Dekontaminationsphase, während derer das Dekontaminationsmittel seine dekontaminierende Wirkung entfaltet. Hierdurch wird verhindert, dass während der vorgenannten Phasen aktiv mit Dekontaminationsmittel angereicherte Raumluft durch die Reinigungseinrichtung gefördert wird, wodurch Dekontaminationsmittel abgebaut würde.

Grundsätzlich wäre es möglich, die Zeit, innerhalb derer die Dekontaminationsmittelkonzentration auf das gewünschte Maß reduziert wird, dadurch zu minimieren, dass eine entsprechend größer dimensionierte Frischluftkonditioniereinrichtung vorgesehen wird. Dies wäre jedoch zum einen mit erhöhten Anlagekosten und Platzbedarf für die größere Anlage verbunden und zudem mit erheblich höheren Energiekosten, da eine wesentlich größere Luftmenge konditioniert, d.h. auf die gewünschte Luftfeuchtigkeit getrocknet und auf die gewünschte Temperatur erhitzt oder gekühlt werden müsste. Um diese Nachteile zu vermeiden, schlägt die Erfindung vor, eine katalytisch wirkende Reinigungseinrichtung, wie sie im Stand der Technik häufig in der Abluftleitung angeordnet ist, bei einer bei einem erfindungsgemäßen Verfahren eingesetzten Dekontaminationsanordnung derart zu platzieren, dass - nach erfolgter Dekontamination - die von dem vorhandenen Dekontaminationsmittel zu befreiende Raumluft im Kreislauf durch diese Reinigungseinrichtung mittels eines Gebläses gefördert wird. Es wird also nicht oder zumindest nicht nur mit Frischluft gespült, sondern mit der zunächst noch mit Dekontaminationsmittel belasteten Raumluft selbst. Diese wird vielfach durch die Reinigungseinrichtung gefördert, wodurch die Dekontaminationsmittelkonzentration von Mal zu Mal abnimmt. Bevorzugt wird zum Fördern der Raumluft im Kreislauf ein Gebläse eingesetzt, welches größer (Leistungsstärker) dimensioniert ist als das Frischluftzufuhrgebläse, um hierdurch die Freispülzeit weiter zu reduzieren. Ganz besonders bevorzugt handelt es sich, wie später noch erläutert werden wird, nicht um ein zusätzlich vorzusehendes Gebläse, sondern um ein in vielen Fällen sowieso bereits vorhandenes Umluftgebläse, welches bei Isolatoranwendungen in der Regel derart ausgelegt ist, dass mit diesem Betrieb des Isolators eine gleichmäßige Luftströmungsgeschwindigkeit von 0,45m/s erreicht werden kann. Die Erfindung reduziert also die Freispülzeit ohne die Notwendigkeit, eine größer Dimensionierte Frischluftkonditioniereinrichtung vorsehen zu müssen, da die im Kreislauf geförderte Raumluft ja bereits konditioniert ist. Gegebenenfalls wird während des Freispülens durch Fördern der Raumluft im Kreislauf durch die Reinigungseinrichtung hindurch zusätzlich Frischluft durch eine gegebenenfalls vorgesehene Frischluftzuführleitung zugeführt, insbesondere um hierdurch einen bestimmten geforderten statischen Luftdruck im zu dekontaminierenden Raum, beispielsweise in einem Isolatorraum aufrecht zu erhalten und/oder um die Freispülzeit weiter zu minimieren. Ggf. wird überschüssige Luft durch eine, bei Bedarf zusätzlich mit einer Reinigungseinrichtung versehene Abluftleitung an die Umgebung abgegeben. Auch ist es, insbesondere bei zu dekontaminierenden Schleusen möglich, während des Freispülens auf eine Frischluftzufuhr und/oder eine Raumluftabfuhr in die Umgebung zu verzichten.

Wie eingangs bereits angedeutet, ist vorgesehen, dass es sich bei der Reinigungseinrichtung um einen Katalysator handelt oder dass die Reinigungseinrichtung einen Katalysator umfasst. Für den Fall der Verwendung von Wasserstoffperoxiddampf als Dekontaminationsmittel hat es sich als bevorzugt herausgestellt, als Katalysator MnO₂ oder Palladium einzusetzen, mit welchem das Dekontaminationsmittel chemisch aufspaltbar ist.

Erfindungsgemäß ist vorgesehen, dass die Reinigungseinrichtung in einem Umlufterzeugerraum angeordnet ist, der insbesondere bei Isolatoren oberhalb des eigentlichen zu dekontaminierenden Raumes angeordnet ist. Der Umlufterzeugerraum zeichnet sich durch ein als Umluftgebläse dienendes Gebläse aus, mit welchem während des bestimmungsgemäßen Betriebes des zu dekontaminierenden Raumes ein Luftstrom, vorzugsweise ein laminarer Luftstrom, insbesondere von oben nach unten erzeugbar ist, vorzugsweise mit einer Strömungsgeschwindigkeit von 0,45m/s. Bei anders aufgebauten Vorrichtungen bzw. zu dekontaminierenden Räumen, beispielsweise bei Schleusen zum Zuführen von Abfüllgut, Pharmazeutika, etc. in einen zu dekontaminierenden Raum ist es möglich, die Reinigungseinrichtung in einen Strömungskanal (Umluftkanal) anzuordnen, vorzugsweise zusammen mit dem Gebläse, wobei der Strömungskanal derart angeordnet ist, dass mit Dekontaminationsmittel versehene Luft aus dem zu dekontaminierenden Raum, insbesondere der Schleuse, angesaugt und die zumindest teilweise bereinigte Luft wieder in den zu dekontaminierenden Raum abgegeben werden kann. Gemäß einer Erfindungsalternative handelt es sich bei dem Strömungskanal bzw. umfasst der Strömungskanal einen Bypass zwischen einer Frischluftleitung zum Zuführen von Frischluft in den zu dekontaminierenden Raum und einer Abluftleitung zum Ablassen von Raumluft an die Umgebung. Weiter bevorzugt ist sowohl die Frischluftleitung als auch die Abluftleitung an jeweils einer Stelle verschließbar, so dass die zu reinigende Raumluft im Kreislauf durch den Bypass förderbar ist, vorzugsweise ohne dass diese Raumluft durch die Frischluftleitung zur Frischluftkonditionseinrichtung und/oder die Abluftleitung in die Umgebung entweichen kann.

Wie eingangs bereits angedeutet, ist es erfindungsgemäß vorgesehen, wenn es sich bei dem zum Einsatz kommenden Gebläse zum Fördern der zu reinigenden Raumluft im Kreislauf durch die Reinigungseinrichtung um ein ohnehin bereits vorhandenes Umluftgebläses zur Erzeugung einer, insbesondere laminaren Luftströmung während des Betriebs des zu dekontaminierenden Raumes handelt. Auf diese Weise muss kein zusätzliches, d.h. separates Gebläse vorgesehen werden, wodurch die Anlagenkosten auf ein Minimum reduziert werden können.

In Weiterbildung des erfindungsgemäßen Verfahrens ist mit Vorteil vorgesehen, dass während der Konditionierungsphase eine Abluftleitung, zumindest zeitweise, vorzugsweise über die größte Zeitspanne, vorzugsweise während der gesamten Zeit, geöffnet ist und dass zum Einblasen von gas- oder dampfförmigen Dekontaminationsmittel nicht Raumluft, sondern, insbesondere konditionierte, Umgebungsluft (Frischluft) und/oder, insbesondere abgefüllte, oder von einem Kompressor erzeugte Druckluft eingesetzt wird. Bevorzugt ist es also, eine so genannte Frisch- oder Druckluftkonditionierung mit Dekontaminationsmittel vorzusehen, wodurch Kondensationserscheinungen minimiert werden können.

Insbesondere für den Fall, dass die Dekontaminationsanordnung einen Isolator, insbesondere für biotechnische oder pharmatechnische Anwendungen umfasst, ist es bevorzugt, wenn dem vorzugsweise als Umluftgebläse dienenden Gebläse in Strömungsrichtung ein Hochleistungsschwebstofffilter, insbesondere ein sogenannter HEPA-Filter oder ein sogenannter ULPA-Filter, nachgeordnet ist, so dass während des Betriebs der Vorrichtung, insbesondere des Isolators, die im Kreislauf beförderte Raumluft vor dem Einströmen in den zu dekontaminierenden Raum, insbesondere einen Manipulatorraum, von gegebenenfalls noch vorhandenen Schwebstoffen gereinigt wird. Ganz besonders bevorzugt ist es, wenn die, insbesondere katalytisch arbeitende, Reinigungseinrichtung in Strömungsrichtung vor dem Hochleistungsschwebstofffilter angeordnet ist, damit aus Richtung des zu dekontaminierenden Raumes Dekontaminationsmittel (von unten) in den Hochleistungsschwebstofffilter eindiffundieren kann. Grundsätzlich ist es hierzu möglich, die Reinigungseinrichtung in Strömungsrichtung vor dem Gebläse anzuordnen, also derart, dass das Gebläse die Raumluft durch die Reinigungseinrichtung saugt. Ganz besonders bevorzugt ist es jedoch wenn die Reinigungseinrichtung zwischen dem Gebläse und dem Hochleistungsschwebstofffilter angeordnet ist, vorzugsweise in einem Strömungskanal, der aus dem Umlufterzeugerraum ausmündet und der einmündet in den zu dekontaminierenden Raum oder in einen Bereich (Zwischenraum) oberhalb des zu dekontaminierenden Raumes, insbesondere einmündet in einen Bereich oberhalb einer später noch zu erläuternden Membran zur Erzeugung einer laminaren, insbesondere von oben nach unten gerichteten Luftströmung. Besonders bevorzugt ist es, wenn die mindestens eine Zuführleitung zum Zuführen von Dekontaminationsmittel derart angeordnet ist, dass die vorerwähnte Reinigungseinrichtung beim Zuführen des Dekontaminationsmittels gebypasst wird, um zu verhindern, dass frisch hinzugeführtes Dekontaminationsmittel vor Abschluss der Dekontaminationsphase eliminiert wird. Vorzugsweise mündet die mindestens eine Zuführleitung oberhalb der ebenfalls vorerwähnten Membran aus, so dass durch diese das zugeführte gas- und/oder dampfförmige Dekontaminationsmittel gleichmäßig verteilt wird bzw. gleichmäßig verteilt den eigentlichen zu dekontaminierenden Raum erreicht. Ganz besonders bevorzugt ist es, wenn nicht nur eine einzige Zuführleitung vorgesehen ist, sondern wenn mehrere die Reinigungseinrichtung bypassend angeordnete Zuführleitungen zur Zuführung von Dekontaminationsmittel vorgesehen sind. Für den Fall, dass sich herausstellen sollte, dass ein Bereich zwischen der Reinigungseinrichtung und dem Hochleistungsschwebstofffilter nicht ausreichend dekontaminiert werden sollte, ist in Weiterbildung der Erfindung vorgesehen, dass, insbesondere mittels einer Abzweigleitung aus mindestens einer Zuführleitung, Dekontaminationsmittel unmittelbar in einen Bereich zwischen der Reinigungseinrichtung und den Hochleistungsschwebstofffilter zugeführt wird.

Besonders zweckmäßig, insbesondere für die Ausbildung der Dekontaminationsanordnung als Isolator ist es, wenn dem zuvor erwähnten Hochleistungsschwebstofffilter in Strömungsrichtung eine Membran, beispielsweise ein Siebdruckgewebe, nachgeordnet ist, die sich bevorzugt unmittelbar über dem eigentlichen zu dekontaminierenden Raum, insbesondere einem Manipulatorraum befindet, und die die von dem Umluftgebläse erzeugte Luftströmung in eine laminare Strömung, vorzugsweise mit einer Strömungsgeschwindigkeit von etwa 0,45m/s umwandelt. Ganz besonders bevorzugt ist es, wenn die mindestens eine Zuführleitung zum Zuführen des Dekontaminationsmittels in einem Bereich oberhalb der vorgenannten Membran endet, ganz besonders bevorzugt unter Umgehung, d.h. Bypassung der Reinigungseinrichtung.

Besonders zweckmäßig ist es, wenn die Dekontaminationsanordnung eine Zuführeinrichtung zum Zuführen des gas- und/oder dampfförmigen Dekontaminationsmittels umfasst. Bevorzugt umfasst diese Zuführeinrichtung Mittel zum Erzeugen des gas- und/oder dampfförmigen Dekontaminationsmittels, insbesondere einen, bevorzugt als Blitzverdampfer ausgebildeten, Verdampfer, mit welchem flüssiges Dekontaminationsmittel zur Erzeugung von Dekontaminationsmitteldampf verdampfbar ist. Die Zuführeinrichtung mündet bevorzugt mit einer Zuführleitung in den zu dekontaminierenden Raum und/oder oberhalb einer gegebenenfalls vorgesehenen, zuvor beschriebenen Membran zur Erzeugung einer laminaren Luftströmung. Bevorzugt ist auch eine Abluftleitung vorgesehen, durch die Luft aus dem zu dekontaminierenden Raum, insbesondere an die Umgebung abführbar ist. Besonders zweckmäßig ist es, wenn während der Konditionierphase, während derer der zu dekontaminierende Raum bis zum Erreichen der gewünschten Konzentration mit Dekontaminationsmittel angereichert wird, die Abluftleitung geöffnet ist und Dekontaminationsmittel, insbesondere dampfförmiges Dekontaminationsmittel mit Hilfe von Druckluft aus Drucktanks oder Druckflaschen oder mit Hilfe von konditionierter Frischluft zugeführt wird. Anders ausgedrückt ist es bevorzugt, wenn eine Druckluft- bzw. Frischluftdekontaminationsmittelkonditionierung durchgeführt wird, also nicht Raumluft abgesaugt und angereichert wird wird, um eine Kondensation von Dekontaminationsmittel im zu dekontaminierenden Raum zumindest weitgehend zu verhindern. Bevorzugt wird während der Konditionierphase ein Ventil und/oder ein Abluftgebläse in der Abluftleitung derart geregelt, dass ein gewünschter Luftdruck in den zu dekontaminierenden Raum aufrecht erhalten wird. Bevorzugt erfolgt die Regelung der Abluftmenge zur Einstellung eines geforderten Raumdruckes zusätzlich oder alternativ auch während des bestimmungsgemäßen Betriebs des zu dekontaminierenden Raumes. Bevorzugt ist auch während der auf die Konditionierungsphase folgenden Dekontaminationsphase die Abluftleitung geöffnet und es wird weiterhin mittels Trägerluft Dekontaminationsmittel, gegebenenfalls in geringer Konzentration, zugeführt.

In Weiterbildung der Erfindung ist mit Vorteil vorgesehen, dass die Dekontaminationsvorrichtung eine Frischluftkonditioniereinrichtung zum Konditionieren, d.h. Temperieren und gegebenenfalls Trocknen von Umgebungsluft (Frischluft) aufweist, wobei die Frischluftkonditioniereinrichtung die Frischluft in den zu dekontaminierenden Raum, vorzugsweise über einen Umlufterzeugerraum fördert. Aufgrund der erfindungsgemäßen Anordnung der Reinigungseinrichtung ist es nicht notwendig zur Erzielung kürzerer Freispülzeiten (Dekontaminationsmittelbereinigungszeiten) eine für den eigentlichen Betrieb überdimensionierte Frischluftkonditioniereinrichtung vorzusehen. Bevorzugt ist die Frischluftkonditioniereinrichtung derart ausgelegt, dass mit dieser mittels konditionierter Luft ein 160facher oder geringerfacher Luftwechsel pro Stunde in dem zu dekontaminierenden Raum und sämtlicher unmittelbar strömungstechnisch mit diesem Raum verbundener Räume möglich ist. Bevorzugt ist die Frischluftkonditioniereinrichtung kleiner dimensioniert, so dass maximal ein 100facher oder maximal ein 50facher oder geringerer Luftwechsel pro Stunde möglich ist.

Wie eingangs erwähnt, ist es aus dem Stand der Technik bekannt, in der Abluftleitung (d.h. nicht im Umluftbetrieb) einen MnO₂-Katalysator zum Reinigen der in die Umgebung abzulassenden Raumluft von Wasserstoffperoxid anzuordnen. Dabei ist üblicherweise MnO₂ als Schüttgut in einem Behältnis aufgenommen, das von der Abluft durchströmt werden kann. Nachteilig ist bei dem Schüttgutkatalysator der vergleichsweise hohe Strömungswiderstand, der eine größere Leistung des Gebläses erfordert, um die geforderte Strömungsgeschwindigkeit von 0,45m/s im Betrieb zu gewährleisten. Um den nachteiligen Strömungswiderstand bei maximaler, wirksamer Oberfläche zu minimieren, ist in Weiterbildung der Erfindung mit Vorteil vorgesehen, dass das Katalysatormaterial nicht als Schüttgut vorliegt, sondern als Beschichtung auf einer Trägerstruktur. Bevorzugt ist die Trägerstruktur mit MnO₂ oder Palladium beschichtet. Ganz besonders bevorzugt weist die Trägerstruktur eine möglichst große Oberfläche auf, um möglichst viel wirksame Katalysatoroberfläche auf möglichst geringem Raum unterbringen zu können. Ganz besonders bevorzugt ist es, wenn die Trägerstruktur von einem, insbesondere offenporigen, Metallschaum, insbesondere einem Aluminiumschaum oder einem Nickelschaum gebildet ist, wobei der Metallschaum mit MnO₂ beschichtet ist, insbesondere elektrolytisch. So eignet sich beispielsweise eine Mangansulfatlösung, mit Hilfe derer elektrolytisch ein Mangatdioxidfilm auf die Trägerstruktur, insbesondere die Metallschaumoberfläche, aufbringbar ist. Ganz besonders bevorzugt besteht der Metallschaum aus 50% Nickel, 22% Eisen, 22% Chrom und 6% Aluminium. Alternativ ist es denkbar, den Metallschaum aus 73% Eisen, 22%Chrom und 6% Aluminium auszubilden. Zur Beschichtung des Metallschaums wird dieser bevorzugt als Anode in einem Elektrolytprozess eingesetzt, wobei aus einem Mangansulfatelektrolysebad Mangandioxid an der Anode bei Anlegen einer Spannung abgeschieden wird.

Wie bereits mehrfach angedeutet, ist es besonders bevorzugt, wenn es sich bei dem dekontaminierenden Raum um einen biotechnischen oder pharmatechnischen Manipulatorraum handelt, also einen Raum, in den, insbesondere über fest mit dem Raum verbundene, Handschuhe eingegriffen werden kann. Zusätzlich oder alternativ handelt es sich bei dem zu dekontaminierenden Raum um einen Handhabungsraum für Pharmazeutika und/oder einen Abfüllraum, umfassend eine Abfülleinrichtung zum Abfüllen von Pharmazeutika. Ganz besonders bevorzugt besteht der zu dekontaminierende Raum aus einer Kombination aus einem Manipulatorraum und einem Abfüll- und/oder Handhabungsraum, wobei der Abfüll- und/oder Handhabungsraum bevorzugt unterhalb des Manipulatorraums angeordnet ist, wobei es noch weiter bevorzugt ist, wenn sich oberhalb des Manipulatorraumes ein Umlufterzeugerraum, umfassend ein Umluftgebläse, befindet.

Ganz besonders zweckmäßig ist es, wenn der zu dekontaminierende Raum ein Volumen von mindestens 0,8dm³, vorzugsweise von mindestens 1m³ oder 5m³, ganz besonders bevorzugt von mindestens 10m³, noch weiter bevorzugt von mindestens 20m³ oder mehr aufweist. Denkbar ist es auch, einen ganzen Gebäuderaum mittels der Erfindung zu dekontaminieren bzw. freizuspülen. Weitere Vorteile, Merkmale und Einzelheiten der Erfindung ergeben sich aus der nachfolgenden Beschreibung bevorzugter Ausführungsbeispiele sowie anhand der Zeichnungen.

Diese zeigen in:
- Fig. 1: in einem Blockschaltbild den grundsätzlichen Aufbau eines möglichen Ausführungsbeispiels einer erfindungsgemäßen Dekontaminationsanordnung,
- Fig. 2: eine als Isolator ausgebildete Dekontaminationsanordnung, umfassend einen Manipulatorraum als zu dekontaminierenden Raum sowie einen ebenfalls zu dekontaminierenden Umlufterzeugerraum oberhalb des Manipulatorraumes, und
- Fig. 3: in einer schematischen Darstellung den grundsätzlichen Aufbau einer alternativen Dekontaminationsanordnung, umfassend beispielsweise eine Schleuse als zu dekontaminierenden Raum.

In den Figuren sind gleiche Elemente und Elemente mit der gleichen Funktion mit den gleichen Bezugszeichen gekennzeichnet.

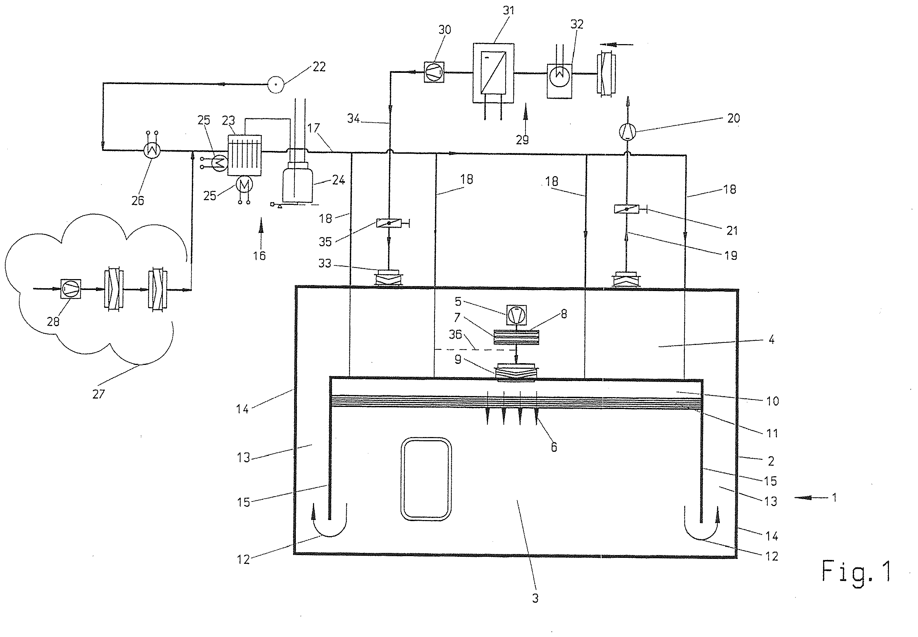

In Fig. 1 ist in einem Blockschaltbild schematisch der Aufbau einer Dekontaminationsanordnung 1 für pharmatechnische Anwendungen gezeigt. Diese umfasst einen Isolator 2 mit einem zu dekontaminierenden Raum 3, der in dem gezeigten Ausführungsbeispiel als Manipulationsraum ausgebildet ist, also mittels Handschuhen von außen durch eine Bedienperson zugänglich ist. Gegebenenfalls kann unterhalb des Manipulatorraums ein Abfüllraum, umfassend eine Abfüllvorrichtung zur Abfüllung von Pharmazeutika vorgesehen sein. Der Manipulatorraum und der Abfüllraum bilden dann eine zu dekontaminierende Raumeinheit (zu dekontaminierender Raum 3).

Oberhalb des zu dekontaminierenden Raumes 3 befindet sich ein Umlufterzeugerraum 4 mit einem als Umluftgebläse dienenden Gebläse 5. Dieses ist derart ausgelegt, dass mit Hilfe des Gebläses 5 eine gleichmäßige Luftströmung 6 von oben nach unten in dem zu dekontaminierenden Raum 3 während des bestimmungsgemäßen Betriebs mit einer Strömungsgeschwindigkeit von etwa 0,45m/s erzeugbar ist. Das Gebläse erzeugt dabei eine turbulente Strömung, die mit einer später noch zu erläuternden Membran, gegebenenfalls in Kombination mit einem Hochleistungsschwebstofffilter in eine laminare Strömung umgewandelt wird.

Dem Gebläse 5 ist in Strömungsrichtung eine Reinigungseinrichtung 7 nachgeordnet. Diese besteht im Wesentlichen aus einem Trägergehäuse für einen Katalysator 8 - in dem gezeigten Ausführungsbeispiel einem MnO₂-Katalysator zur Aufspaltung von Wasserstoffperoxiddampf. Der Katalysator 8 ist gebildet von einem mittels MnO₂ beschichteten Metallschaum, so dass bei Durchströmung des Katalysators 8 ein geringer Strömungswiderstand anfällt und dass der Katalysator 8 möglichst kleinvolumig baut.

Der Reinigungseinrichtung 7 ist in Strömungsrichtung ein Hochleistungsschwebstofffilter 9, beispielsweise ein HEPA-Filter oder ein ULPA-Filter, nachgeordnet. Der von dem Gebläse erzeugte Luftstrom gelangt nach dem Hochleistungsschwebstofffilter 9 in einen Zwischenraum 10, der unten von einer Membran 11, beispielsweise aus einem Siebdruckgewebe, begrenzt ist. Die Membran 11 erzeugt aus dem Luftstrom die laminare Luftströmung 6, welche die vorerwähnte Geschwindigkeit von 0,45m/s im Betrieb aufweist. Die Luft strömt also in dem zu dekontaminierenden Raum 3 nach unten und dort in Pfeilrichtung 12 nach oben in Strömungskanäle 13, die ausgebildet sind zwischen, beispielsweise von Glasplatten gebildeten Isolatorwänden 14 und inneren, parallel zu den Isolatorwänden 14 mit Abstand zu diesen angeordneten transparenten, vorzugsweise aus Glas ausgebildeten, Platten 15. Durch diese Strömungskanäle 13 kann die von dem Gebläse 5 geförderte Raumluft wieder in den Umlufterzeugerraum 4 strömen, aus dem die Luft dann wieder mittels des Gebläses 5 angesaugt wird. Die Raumluft wird also während des Betriebs des Gebläses 5 im Kreislauf durch die Reinigungseinrichtung 7 gefördert.

Um den Isolator 2, genauer den zu kontaminierenden Raum bestimmungsgemäß verwenden bzw. betreiben zu können, muss dieser zunächst dekontaminiert werden. Hierzu ist eine Zuführeinrichtung 16 vorgesehen, mit Hilfe derer gas- und/oder dampfförmiges Dekontaminationsmittel, insbesondere gemischt mit Trägerluft, zugeführt werden kann. Die Zuführeinrichtung 16 umfasst eine zentrale Versorgungsleitung 17, die in dem gezeigten Ausführungsbeispiel als Verteilerleitung dient. Von der Versorgungsleitung 17 gehen in dem gezeigten Ausführungsbeispiel insgesamt vier Zuführleitungen 18 ab, die in den Zwischenraum 10 oberhalb der Membran 11 unter Umgehung der Reinigungseinrichtung 7 ausmünden. Das Dekontaminationsmittel verteilt sich also während der Konditionierungsphase, im Zwischenraum 10, fällt dann nach unten ab durch die Membran 6 hindurch und kann dann in Pfeilrichtung 12 durch die Strömungskanäle 13 nach oben zu einer Abluftleitung 19 strömen, die während der Konditionierungsphase in der der Isolator mit Dekontaminationsmittel angereichert wird geöffnet ist. In der Abluftleitung 19 kann ein Abluftgebläse 20 vorgesehen werden, mit dem gezielt Raumluft aus dem Isolator 2 abgesaugt werden kann. Der Raumluftdruck wird geregelt über das Abluftgebläse 20 in der Abluftleitung 19. Da durch die Abluftleitung 19 Raumluft entweichen kann, verteilt sich das Dekontaminationsmittel im eigentlichen zu dekontaminierenden Raum 3 und auch im Umlufterzeugerraum 4 gleichmäßig. Dadurch, dass während der Konditionierungsphase Raumluft durch die Abluftleitung 19, gegebenenfalls unter Einsatz des Abluftgebläses 20 abgelassen wird, werden Kondensationserscheinungen des Dekontaminationsmittels an den Umfangswänden vermieden.

Die Zuführeinrichtung 16 für Dekontaminationsmittel umfasst in dem gezeigten Ausführungsbeispiel eine Druckluftquelle 22 für Trägerluft, beispielsweise eine Druckluftflasche und einen Verdampfer 23, mit Hilfe dessen Dekontaminationsmittel, hier Wasserstoffperoxid, welches aus einem Vorratsbehälter 24 zugeführt wird, verdampft werden kann. Der Verdampfer 23 verfügt über entsprechende Heizungen 25. Dem Verdampfer 23 ist eine Vorheizung 26 vorgeschaltet, um die Druckluft (Trägerluft) geeignet temperieren zu können.

Die Zuführeinrichtung 16 kann alternativ beispielsweise wie unten dargestellt und wie mit dem Bezugszeichen 27 gekennzeichnet ausgebildet werden. Wesentlicher Unterschied ist hier, dass die Zuführeinrichtung 27 im Gegensatz zu der oben dargestellten Zuführeinrichtung 27 ein Zuführgebläse 28 zum Zuführen von Frischluft aufweist. Diese sollte geeignet konditioniert, d.h. getrocknet und temperiert werden. Der Verdampfer 23 kann auch einer später noch zu erläuternden Frischluftkonditioniereinrichtung nachgeschaltet werden, entweder unmittelbar angeschlossen an die Frischluftleitung oder im Bypass hierzu. Hierdurch kann auf eine separate Konditioniereinrichtung verzichtet werden.

Unabhängig von der Trägerluft (Druckluft oder konditionierte Frischluft) wird jedenfalls zunächst Dekontaminationsmittel während einer Konditionierungsphase, während der die Abluftleitung 19 geöffnet ist, zugeführt. Nach Beendigung der auf die Konditionierungsphase, in der die gewünschte Dekontaminationsmittelkonzentration eingestellt bzw. erreicht wird, folgenden Dekontaminationsphase wird die Zuführeinrichtung 16 bzw. 27 abgeschaltet bzw. abgekoppelt und der zu dekontaminierende Raum 3 wird von Dekontaminationsmittel befreit. Hierzu wird das während der Konditionierungsphase und der Dekontaminierungsphase ausgeschaltete Gebläse 5 angeschaltet, so dass die Raumluft 3 im Kreislauf durch die Reinigungseinrichtung 7 gefördert wird, wodurch die Dekontaminationsmittelkonzentration durch katalytische Wirkung des eingesetzten Katalysators 8 reduziert wird. Nach Erreichen der gewünschten Minimalkonzentration, beispielsweise 1ppm; 0,5ppm oder weniger kann zum bestimmungsgemäßen Betrieb des zu dekontaminierenden Raums 3 übergegangen werden.

In Fig. 1 ist eine Frischluftkonditionierungseinrichtung 29 zu erkennen, umfassend ein Frischluftgebläse 30, das derart ausgelegt ist, dass mit diesem ein um 150facher Luftwechsel pro Stunde im Isolator 2 möglich ist. Die Frischluft kann mittels einer Kühleinrichtung 31 gekühlt oder mit einer Heizeinrichtung 32 erwärmt werden. Bevorzugt wird die Luft getrocknet. Die zugeführte Frischluft strömt über ein als HEPA- oder ULPA-Filter ausgebildetes Filter 33 in den Umlufterzeugerraum 4. Zu erkennen ist, dass die Frischluftleitung 34, die zum Filter 33 führt, mittels eines Ventils 35 absperrbar ist. Während des bestimmungsgemäßen Gebrauchs wird über die Frischluftkonditioniereinrichtung 29 Frischluft in das System zugeführt, insbesondere um einen unvermeidlichen Druckverlust während des Betriebs, gegebenenfalls vorhandene Leckagestellen in einem gegebenenfalls vorgesehenen Abfüllraum ausgleichen zu können. Leckagestellen sind insbesondere dann unvermeidlich, wenn aus dem zu dekontaminierenden Raum 3 Güter, beispielsweise gefüllte Pharmazeutikabehältnisse, meist kontinuierlich, herausgeführt werden müssen. Der gewünschte Druck im Isolator 1 wird bevorzugt über das Abluftgebläse 20 in der Abluftleitung 19 geregelt.

Es ist nicht notwendig, während der Freispülphase (Umluftphase), also während der Reinigung der Raumluft, Frischluft über die Frischluftleitung 34 zuzuführen. Der Freispülprozess kann jedoch durch diese Maßnahme noch weiter beschleunigt werden.

Um die Strömungsgeschwindigkeit von 0,45m/s zu gewährleisten, ist das als Umluftgebläse während des Betriebs dienende Gebläse größer dimensioniert (insbesondere mindestens doppelt so groß), in dem gezeigten Ausführungsbeispiel fünffach größer d.h. fünffach leistungsstärker dimensioniert als das Frischluftgebläse 30, so dass ein wesentlich größerer Volumenstrom, hier ein fünffach größerer Volumenstrom, durch die Reinigungseinrichtung 7 gefördert werden kann, als Frischluft durch die Frischluftleitung 34 zugeführt werden könnte.

In Fig. 1 ist eine fakultative Stichleitung 36 zu erkennen, die mindestens eine Zuführleitung 18 für Dekontaminationsmittel mit einem Bereich des Strömungskanals zwischen der Reinigungseinrichtung 7 und dem Hochleistungsschwebstofffilter 9 verbindet, um sicherzustellen, dass auch dieser Zwischenbereich ausreichend dekontaminiert wird.

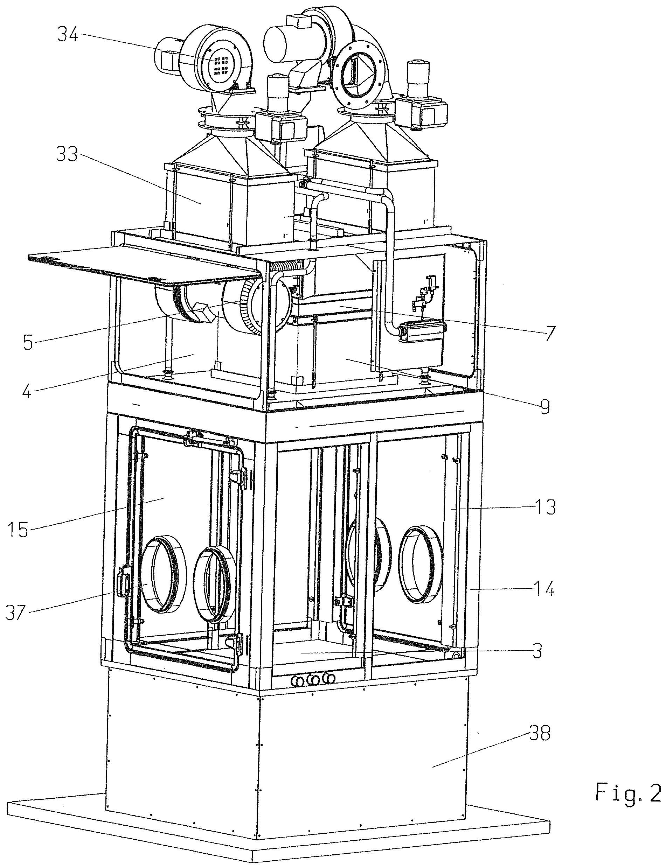

Fig. 2 zeigt einen möglichen Aufbau eines Isolators 2. Zu erkennen ist der als Manipulationsraum ausgebildete, zu dekontaminierende Raum 3, der über Öffnungen 37 von Hand mittels nicht dargestellten Handschuhen zugänglich ist, die am Isolator fixiert sind und die die Öffnungen 37 abdichten. Hinter den aus Glas ausgebildeten Isolatorwänden 14 befinden sich als Glasplatten ausgebildete Platten 15 zur Ausbildung von Strömungskanälen 13, durch die Raumluft aus den zu dekontaminierenden Raum in den oberhalb angeordneten Umlufterzeugerraum 4 strömen kann. In diesen mündet eine Frischluftleitung 34 über ein Filter 33. In dem Umlufterzeugerraum 4 befindet sich das Hochleistungsschwebstofffilter 9, oberhalb dessen die einen MnO₂-Katalysator umfassende Reinigungseinrichtung 7 angeordnet ist und zwar unterhalb eines als Umlufterzeugergebläse ausgebildeten Gebläses 5, welches Raumluft aus dem Umlufterzeugerraum 4 ansaugt und somit im Kreislauf durch die Reinigungseinrichtung 7 fördern kann.

In dem gezeigten Ausführungsbeispiel sitzt der Manipulatorraum auf einem schematisch angedeuteten Gestell 38. Anstelle des Gestells 38 kann ein nach oben offener Abfüllraum, insbesondere beinhaltend eine Abfüllmaschine, oder ein sonstiger Raum vorgesehen werden, der einen Teil des zu dekontaminierenden Raums 3 bildet.

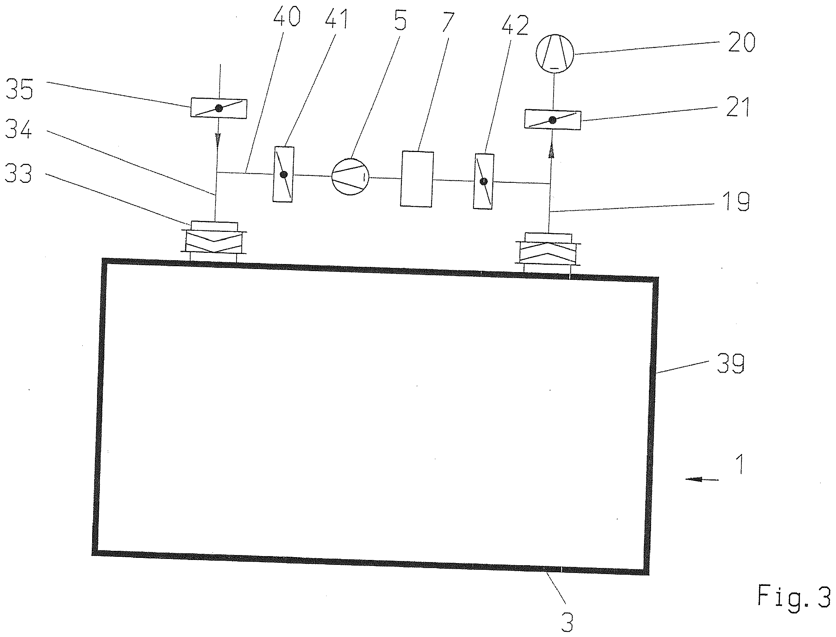

Fig. 3 zeigt in einer schematischen Darstellung den Aufbau einer alternativen Dekontaminationsanordnung 1, die in dem gezeigten Ausführungsbeispiel eine Schleuse 39 als zu dekontaminierenden Raum 3 umfasst. In den zu dekontaminierenden Raum 3 mündet über ein als HEPA- oder ULPA-Filter ausgebildetes Filter 33 eine Frischluftleitung 34, der eine nicht dargestellte Frischluftkonditioniereinrichtung vorgeschaltet ist. Aus dem zu dekontaminierenden Raum 3 mündet eine mittels eines Ventils 21 verschließbare Abluftleitung 19 aus. Dem Filter 33 ist in der Frischluftleitung 34 ein als Absperrventil ausgebildetes Ventil 35 vorgeschaltet. In Strömungsrichtung nach dem Ventil 35 und vor dem Ventil 21 ist die Frischluftleitung 34 über eine als Bypass ausgebildete Umluftleitung 40 verbunden, in der ein Gebläse 5 sowie eine hier katalytisch arbeitende Reinigungseinrichtung 7 angeordnet ist. Die Umluftleitung 40 ist mit je einem Ventil 42, 41 in Strömungsrichtung vor und nach der Reinigungseinrichtung 7 absperrbar. Während einer Dekontaminationsphase sind die Ventile 41, 42 geschlossen und es wird, gegebenenfalls über die Frischluftleitung 34 oder eine separate, nicht dargestellte, Zuführleitung Dekontaminationsmittel zugeführt. Hierbei ist das Ventil 21 geöffnet. Gegebenenfalls wird zusätzlich ein fakultativ vorgesehenes Abluftgebläse 20 betrieben. Nach der auf die Dekontaminationsphase folgenden Reinigungsphase wird zumindest das Ventil 21, bevorzugt auch das Ventil 35, geschlossen, und die Raumluft aus dem zu dekontaminierenden Raum 3 wird im Kreislauf durch die Umluftleitung 40 und damit durch die Reinigungseinrichtung 7 gefördert, um auf diesem Weg Dekontaminationsmittel aus der Raumluft zu entziehen, bevorzugt indem dieses chemisch aufgespaltet wird.

## Patentansprüche

1. Verfahren zum Entfernen von gas- und/oder dampfförmigen Dekontaminationsmittel, insbesondere von Wasserstoffperoxiddampf, aus einem zu dekontaminierenden Raum (3) mittels einer Dekontaminationsanordnung, insbesondere für pharmatechnische Anwendungen, umfassend einen zu dekontaminierenden Raum (3), insbesondere einen Isolatorraum sowie eine zum Entziehen von gas- und/oder dampfförmigen Dekontaminationsmittel, insbesondere Wasserstoffperoxid, aus der Raumluft ausgebildete Reinigungseinrichtung (7), wobei die Reinigungseinrichtung (7) derart angeordnet ist, dass die Raumluft durch diese im Kreislauf mittels mindestens eines Gebläses (5) förderbar ist, und wobei die Reinigungseinrichtung (7) einen Katalysator (8), insbesondere MnO₂ oder Palladium, zum chemischen Aufspalten des Dekontaminationsmittels umfasst oder aus einem derartigen Katalysator (8) besteht,
und wobei die Reinigungseinrichtung (7)
in einem oberhalb des zu dekontaminierenden Raums (3) angeordneten Umlufterzeugerraum (4)
oder
in einer als Bypass zwischen einer verschließbaren Frischluftleitung (34) und einer verschließbaren Abluftleitung (19) ausgebildeten Umluftleitung (40)
angeordnet ist,
und wobei die Raumluft des zu dekontaminierenden Raumes (3) mittels des Gebläses (5) im Kreislauf durch die Reinigungseinrichtung (7) gefördert wird, mit der das Dekontaminationsmittel, insbesondere katalytisch, aus der Raumluft entfernt wird,
**dadurch gekennzeichnet,**
**dass** das Gebläse (5), zumindest über den größten Zeitabschnitt, während einer Konditionierungsphase, in der Dekontaminationsmittel zugeführt wird und/oder während einer Einwirkphase während derer das Dekontaminationsmittel seine dekontaminierende Wirkung entfaltet ausgeschaltet wird oder bleibt.

2. Verfahren nach Anspruch 1,
**dadurch gekennzeichnet,**
**dass** während der Konditionierungsphase und/oder während einer Dekontaminationsphase eine Abluftleitung (19) zum Abführen von mit Dekontaminationsmittel angereicherter Raumluft aus dem zu dekontaminierenden Raum geöffnet ist und/oder dass während Raumluft im Kreislauf durch die Reinigungseinrichtung (7) gefördert wird zusätzlich Frischluft über eine Frischluftleitung (34) zugeführt wird.

3. Verfahren nach einem der Ansprüche 1 oder 2,
**dadurch gekennzeichnet,**
**dass** das Gebläse (5) ein Umluftgebläse zur Erzeugung einer Umluftströmung während des Betriebs des zu dekontaminierenden Raumes (3) ist.

4. Verfahren nach einem der vorhergehenden Ansprüche,
**dadurch gekennzeichnet,**
**dass** dem Gebläse (5) in Strömungsrichtung ein Hochleistungsschwebstofffilter(9), insbesondere einem ULPA-Filter oder einem HEPA-Filter, nachgeordnet ist, und dass die Reinigungseinrichtung (7) in Strömungsrichtung vor dem Hochleistungsschwebstofffilter (9), insbesondere zwischen dem Gebläse (5) und dem Schwebstofffilter angeordnet ist.

5. Verfahren nach Ansprüche 4,
**dadurch gekennzeichnet,**
**dass** dem Hochleistungsschwebstofffilter (9) in Strömungsrichtung eine zum Erzeugen einer laminaren Strömung, insbesondere mit einer Strömungsgeschwindigkeit von etwa 0,45 m/s, ausgebildete Membran (11) nachgeordnet ist und dass Dekontaminationsmittel in einen Bereich oberhalb der Membran (11) zuführbar ist.

6. Verfahren nach einem der vorhergehenden Ansprüche,
**dadurch gekennzeichnet,**
**dass** eine, vorzugsweise einen Verdampfer (23) umfassende Zuführeinrichtung (16) zum Zuführen des gas- und/oder dampfförmigen Dekontaminationsmittels vorgesehen ist und dass eine Abluftleitung (19) zum Abführen von überschüssigem Dekontaminationsmittel während einer Konditionierphase vorgesehen ist.

7. Verfahren nach einem der vorhergehenden Ansprüche,
**dadurch gekennzeichnet,**
**dass** umfassend eine Frischluftkonditioniereinrichtung (29) zum Konditionieren und Zuführen von Frischluft, wobei die Frischluftkonditioniereinrichtung (29) derart ausgelegt ist, dass mit dieser eine Frischluftvolumenstrom zuführbar ist, um einen weniger als 160fachen oder weniger als 100fachen oder weniger als 50fachen Luftwechsel in dem zu dekontaminierenden Raum (3) und aller strömungstechnisch mit diesem Raum verbundener Räume zu ermöglichen.

8. Verfahren nach einem der vorhergehenden Ansprüche,
**dadurch gekennzeichnet,**
**dass** die Reinigungseinrichtung (7) eine mit einer reaktiven oder katalytisch wirksamen Substanz, insbesondere MnO₂ oder Palladium, beschichtete Trägerstruktur, insbesondere eine offenporige Metallschaumstruktur oder eine Gitterstruktur umfasst oder daraus besteht.

9. Verfahren nach Anspruch 8,
**dadurch gekennzeichnet,**
**dass** die Substanz elektrolytisch abgeschieden ist.

10. Verfahren nach einem der vorhergehenden Ansprüche,
**dadurch gekennzeichnet,**
**dass** der zu dekontaminierende Raum (3) als biologischer oder pharmatechnischer Manipulatorraum oder als Handhabungsraum für Pharamzeutika oder als Abfüllraum mit einer Abfülleinrichtung zum Abfüllen von Pharamzeutika ausgebildet ist oder mindestens einen solchen Raum umfasst.

11. Verfahren nach einem der vorhergehenden Ansprüche,
**dadurch gekennzeichnet,**
**dass** der zu dekontaminierende Raum (3) ein Volumen von mindestens 0,8dm³, vorzugsweise von mindestens 1m³ oder 5m³, bevorzugt von mindestens 10m³, besonderes bevorzugt von mindestens 20m³ oder mindestens 100m³ aufweist.

## Claims

1. A method for removing a gaseous and/or vaporous decontaminant, in particular hydrogen peroxide vapor, from a space (3) to be decontaminated by means of a decontamination assembly, in particular for pharmaceutical applications, including a space (3) to be decontaminated, in particular an isolator space, as well as a purification device (7) configured to withdraw a gaseous and/or vaporous decontaminant, in particular hydrogen peroxide, from the ambient air, wherein the cleaning device (7) is arranged in such a way that the ambient air can be conveyed by at least one blower (5) through this device (7) in a circulating manner, and wherein the purification device (7) includes a catalyst (8), in particular MnO₂ or palladium, for chemically cleaving the decontaminant, or consists of such a catalyst (8),
and wherein the purification device (7)
is arranged in a recirculating air-producing space (4) arranged above the space (3) to be decontaminated
or
in a recirculating-air line (40) configured as a bypass between a closable fresh air line (34) and a closable waste air line (19),
and wherein the ambient air of the space to be decontaminated (3) is conveyed, by means of the blower (5), so as to circulate through the purification device (7), said apparatus removing, in particular catalytically removing, the decontaminant from the ambient air,
**characterized in that**
the blower (5) is or remains turned off at least for the largest time period in a conditioning phase, during which decontaminant is fed and/or during an exposure phase, during which the decontaminant takes effect.

2. The method according to claim 1,
**characterized in that**
during the conditioning phase and/or during a decontamination phase, a waste air line (19) for discharging ambient air rich of decontaminant from the space to be decontaminated is open and/or that additionally fresh air is fed via a fresh air line (34) while ambient air is conveyed in the circulation system by means of the purification device (7).

3. The method according to one of claims 1 or 2,
**characterized in that**
the blower (5) is a recirculating air blower for producing a recirculating air stream during operation of the space (3) to be decontaminated.

4. The method according to one of the preceding claims,
**characterized in that**
the blower (5) has a high-performance floating particle filter (9), in particular an ULPA filter or a HEPA filter, and **in that** the purification device (7) is arranged upstream in the flow direction of the high-performance floating particle filter (9), in particular between the blower (5) and the floating particle filter.

5. The method according to claim 4,
**characterized in that**
the high-performance floating particle filter (9) has a membrane (11) configured to generate a laminar flow, in particular with a flow rate of about 0.45 m/sec, and **in that** decontaminant can be fed into a region above this diaphragm (11).

6. The method according to one of the preceding claims,
**characterized in that**
a feeding device (16), which preferably includes a vaporizer (23), is provided for feeding the gaseous and/or vaporous decontaminant, and that a waste air line (19) is provided in order to discharge excess decontaminant during a conditioning phase.

7. The method according to one of the preceding claims,
**characterized in that**
including a fresh-air conditioning device (29) for conditioning and feeding fresh air, wherein this fresh-air conditioning device (29) is adapted such that a fresh air volume flow by this device can be fed in order to enable an air exchange of less than 160 times, or less than 100 times, or less than 50 times in the space to be decontaminated (3) and of all spaces in fluidic communication with this space.

8. The method according to one of the preceding claims,
**characterized in that**
the cleaning device (7) includes or consists of a carrier structure coated with a substance acting reactively or catalytically, in particular MnO₂ or palladium, in particular an open-pore metal foam structure or a grid structure.

9. The method according to claim 8,
**characterized in that**
the substance is deposited by means of electrolytes.

10. The method according to one of the preceding claims,
**characterized in that**
the space to be decontaminated (3) is formed as a biological or pharmaceutical manipulating space or as a management space for pharmaceutics or as a filling space with a filling apparatus for filling of pharmaceuticals, or comprises at least one such space.

11. The method according to one of the preceding claims,
**characterized in that**
the space to be decontaminated (3) has a volume of at least 0,8 dm³, preferably at least 1 m³ or 5 m³, preferably at least 10 m³, particularly preferably at least 20 m³ or at least 100 m³.

## Revendications

1. Procédé d'élimination d'agents de décontamination gazeux et/ou sous forme de vapeur, en particulier de vapeur de peroxyde d'hydrogène, hors d'un espace à décontaminer (3) au moyen d'un dispositif de décontamination, en particulier pour des applications pharmaco techniques, comprenant un espace à décontaminer (3), en particulier un espace d'isolateur ainsi qu'un dispositif d'épuration (7) réalisé pour extraire un agent de décontamination gazeux et/ou sous forme de vapeur, en particulier du peroxyde d'hydrogène, hors de l'air ambiant, le dispositif d'épuration (7) étant disposé de telle sorte que l'air ambiant puisse être transporté par celui-ci en circuit au moyen d'au moins une soufflante (5), et le dispositif d'épuration (7) comprenant un catalyseur (8), en particulier du MnO₂ ou du palladium, pour la séparation chimique de l'agent de décontamination ou se composant d'un tel catalyseur (8),
et le dispositif d'épuration (7) étant disposé dans un espace générateur de circulation d'air (4) disposé au-dessus de l'espace à décontaminer (3),
ou dans une conduite de circulation d'air (40) réalisée sous forme de dérivation entre une conduite d'air frais (34) pouvant être fermée et une conduite d'évacuation d'air (19) pouvant être fermée,
et l'air ambiant de l'espace à décontaminer (3) étant transporté au moyen de la soufflante (5) en circuit à travers le dispositif d'épuration (7) avec lequel l'agent de décontamination est éliminé, notamment sous forme catalytique, hors de l'air ambiant, **caractérisé en ce que** la soufflante (5) est ou reste déconnectée au moins pendant le plus grand intervalle de temps, pendant une phase de conditionnement durant laquelle l'agent de décontamination est acheminé, et/ou pendant une phase d'action durant laquelle l'agent de décontamination déploie son effet de décontamination.

2. Procédé selon la revendication 1, **caractérisé en ce que** pendant la phase de conditionnement et/ou pendant une phase de décontamination, une conduite d'évacuation d'air (19) est ouverte pour évacuer l'air ambiant enrichi en agent de décontamination hors de l'espace à décontaminer et/ou **en ce que** pendant que l'air ambiant est transporté en circuit à travers le dispositif d'épuration (7), de l'air frais est en outre acheminé par le biais d'une conduite d'air frais (34).

3. Procédé selon l'une quelconque des revendications 1 ou 2, **caractérisé en ce que** la soufflante (5) est une soufflante de circulation d'air pour générer un écoulement d'air en circulation pendant le fonctionnement de l'espace à décontaminer (3).

4. Procédé selon l'une quelconque des revendications précédentes, **caractérisé en ce que** la soufflante (5) est suivie, dans le sens de l'écoulement, d'un filtre à particules en suspension de grande puissance (9), en particulier un filtre ULPA ou un filtre HEPA, et **en ce que** le dispositif d'épuration (7) est disposé dans le sens de l'écoulement avant le filtre à particules en suspension de grande puissance (9), en particulier entre la soufflante (5) et le filtre à particules en suspension.

5. Procédé selon la revendication 4,**caractérisé en ce que** le filtre à particules en suspension de grande puissance (9) est suivi, dans le sens de l'écoulement, d'une membrane (11) réalisée pour générer un écoulement laminaire, en particulier à une vitesse d'écoulement d'environ 0,45 m/s, et **en ce que** l'agent de décontamination peut être acheminé dans une région au-dessus de la membrane (11) .

6. Procédé selon l'une quelconque des revendications précédentes, **caractérisé en ce qu'**un dispositif d'acheminement (16), comprenant de préférence un évaporateur (23), est prévu pour acheminer l'agent de décontamination gazeux et/ou sous forme de vapeur et **en ce qu'**une conduite d'évacuation d'air (19) est prévue pour évacuer l'agent de décontamination en excès pendant une phase de conditionnement.

7. Procédé selon l'une quelconque des revendications précédentes, **caractérisé en ce qu'**il comprend un dispositif de conditionnement d'air frais (29) pour conditionner et acheminer de l'air frais, le dispositif de conditionnement d'air frais (29) étant conçu de telle sorte qu'un débit d'air frais puisse être acheminé au moyen de celui-ci afin de permettre un changement d'air inférieur à 160 fois, ou inférieur à 100 fois ou inférieur à 50 fois, dans l'espace à décontaminer (3) et dans tous les espaces liés fluidiquement à cet espace.

8. Procédé selon l'une quelconque des revendications précédentes, **caractérisé en ce que** le dispositif d'épuration (7) comprend ou se compose d'une structure porteuse revêtue d'une substance réactive ou à action catalytique, en particulier du MnO₂ ou du palladium, en particulier d'une structure à mousse métallique à pores ouverts ou d'une structure en treillis.

9. Procédé selon la revendication 8, **caractérisé en ce que** la substance est déposée par voie électrolytique.

10. Procédé selon l'une quelconque des revendications précédentes, **caractérisé en ce que** l'espace à décontaminer (3) est réalisé en tant qu'espace de manipulation biologique ou pharmaco technique, ou en tant qu'espace de manipulation pour produits pharmaceutiques ou en tant qu'espace de remplissage avec un dispositif de remplissage pour le remplissage de produits pharmaceutiques, ou comprend au moins un tel espace.

11. Procédé selon l'une quelconque des revendications précédentes, **caractérisé en ce que** l'espace à décontaminer (3) présente un volume d'au moins 0,8 dm³, de préférence d'au moins 1 m³ ou de 5 m³, de préférence d'au moins 10 m³, particulièrement préférablement d'au moins 20 m³ ou d'au moins 100 m³.
